# EUROPEAN PATENT SPECIFICATION

(11) **EP 3 197 346 B1**
(45) Date of publication and mention of the grant of the patent: **26.02.2020**
(21) Application number: 15845374.6
(22) Date of filing: 22.09.2015
(51) Int. Cl.: G01N 33/68, G01N 33/564, G01N 33/577

(54) **USE OF 5-METHOXYTRYPTOPHAN AS DIAGNOSTIC AGENT OF INFLAMMATORY DISEASES**
VERWENDUNG VON 5-METHOXYTRYPTOPHAN ALS DIAGNOSEMITTEL VON ENTZÜNDUNGSKRANKHEITEN
UTILISATION DE 5-MÉTHOXYTRYPTOPHANE COMME AGENT DE DIAGNOSTIC DE MALADIES INFLAMMATOIRES

(30) Priority: 22.09.2014 US 201462053760 P
(43) Date of publication of application: 02.08.2017
(73) Proprietor: National Health Research Institutes, Zhunan Township, Miaoli County 350 (TW)
(72) Inventor: WU, Kenneth Kun-Yu, Zhunan Miaoli County 350 (TW); KUO, Cheng-Chin, Zhunan Miaoli County 350 (TW)
(74) Representative: Viering, Jentschura & Partner mbB Patent- und Rechtsanwälte
(86) International application number: PCT/US2015/051482
(87) International publication number: WO 2016/049044

(56) References cited:
- WO-A1-2007/054348
- US-A- 4 762 781
- US-A1- 2005 214 859
- US-A1- 2007 167 474
- US-A1- 2012 004 854
- US-A1- 2012 004 854
- US-A1- 2014 113 833
- HUEI-HSUAN CHENG ET AL: "Quiescent and Proliferative Fibroblasts Exhibit Differential p300 HAT Activation through Control of 5-Methoxytryptophan Production", PLOS ONE, vol. 9, no. 2, 11 February 2014 (2014-02-11), page e88507, XP055446487, DOI: 10.1371/journal.pone.0088507
- H.-H. CHENG ET AL: "Control of cyclooxygenase-2 expression and tumorigenesis by endogenous 5-methoxytryptophan", PROCEEDINGS NATIONAL ACADEMY OF SCIENCES PNAS, vol. 109, no. 33, 31 July 2012 (2012-07-31), pages 13231-13236, XP055446490, US ISSN: 0027-8424, DOI: 10.1073/pnas.1209919109
- Wang Yifu ET AL: "5-methoxytryptophan is a circulating anti-inflammatory molecule that controls lipopolysaccharide-induced systemic inflammation and sepsis (IRM11P.622)", The Journal of Immunology May 1, 2015, 194 (1 Supplement) 132.1;, 1 May 2015 (2015-05-01), page 132.1, XP055447758, Retrieved from the Internet: URL:http://www.jimmunol.org/content/194/1_ Supplement/132.1.short [retrieved on 2018-02-05]
- HSU-FENG HU ET AL: "Control of lupus by novel tryptophan metabolite 5-methoxytryptophan. (THER5P.907)", THE JOURNAL OF IMMUNOLOGY, vol. 194, no. 1 suppl, 1 May 2015 (2015-05-01), page 139.9, XP055447766,
- GEFFARD M ET AL: "ANTISERA AGAINST THE INDOLEALKYLAMINES: TRYPTOPHAN, 5-HYDROXYTRYPTOPHAN, 5-HYDROXYTRYPTAMINE, 5-METHOXYTRYPTOPHAN, AND 5-METHOXYTRYPTAMINE TESTED BY AN ENZYME-LINKED IMMUNOSORBENT ASSAY METHOD", JOURNAL OF NEUROCHEMISTRY, WILEY INTERSCIENCE, NEW YORK, NY, US, vol. 44, no. 4, 1 April 1985 (1985-04-01), pages 1221-1228, XP000573118, ISSN: 0022-3042, DOI: 10.1111/J.1471-4159.1985.TB08747.X
- VIELMA JOSÉ RAMÓN ET AL: "Effects of melatonin on oxidative stress, and resistance to bacterial, parasitic, and viral infections: A review", ACTA TROPICA, vol. 137, 6 May 2014 (2014-05-06), pages 31-38, XP028877426, ISSN: 0001-706X, DOI: 10.1016/J.ACTATROPICA.2014.04.021
- KENNETH K WU ET AL: "5-methoxyindole metabolites of L-tryptophan: control of COX-2 expression, inflammation and tumorigenesis", JOURNAL OF BIOMEDICAL SCIENCE, KLUWER ACADEMIC PUBLISHERS, DO, vol. 21, no. 1, 3 March 2014 (2014-03-03), page 17, XP021179781, ISSN: 1423-0127, DOI: 10.1186/1423-0127-21-17

## Description

### BACKGROUND OF THE INVENTION

### Technical Field of the Invention

The present invention relates to a diagnostic method for inflammatory diseases, and the diagnostic kit used in the method. In particular, the present invention relates to a diagnostic method of inflammatory diseases by using 5-methoxytryptophan (5-MTP) as a diagnostic biomarker. The present invention also specially relates to a highly specific competitive ELISA to measure 5-MTP level in human serum for gauging occurrence and severity of inflammatory diseases, such as sepsis and systemic lupus erythematosus (SLE).

### Background

Inflammation is an old concept with new definitions. Its molecular and cellular mechanisms have advanced rapidly which have revolutionized its biomedical significances and applications. Inflammation is caused by excessive and inappropriate innate immune system activity such as an overwhelming activation of COX-2- and Toll-like receptors (TLRs)-signaling; the process is now considered as a fundamental process in many important human diseases and health issues such as sepsis, Systemic Lupus Erythematosus (SLE), cardiovascular diseases, metabolic syndrome, cancer, septicemia and diverse inflammatory joint, gastrointestinal and renal diseases. These diseases affect tens of millions of worldwide health, social and economic impact of the high cost of health care burden. In terms of 2010 for example sepsis spends about 5 billion U.S. dollars a year. Cellular and molecular factors responsible for inflammation in the diverse health problems and diseases are complex and not necessarily identical. Cytokines, chemokines and small molecular mediators such as eicosanoids, nitric oxide and other lipid mediators are emerging as important targets for control of various inflammatory disorders. On the other hand, biomarkers have been broadly used to aid in the diagnosis, prognostication or therapy of many medical conditions (Ridker, Nutrition reviews 65:S253-259, 2007). They are also important for improving patient treatment and new drug discovery.

Cytokines including TNF, IL-1β and IL-6 that mediate the initial response of the innate immune system to injury or infection. These cytokines can activate endothelial cells, attracting circulating polymorphonuclear leukocytes (PMNs) to the site. They also enter the circulation, causing fever and other systemic symptoms. Furthermore, certain cytokines such as IL-6 enhance the liver's production of the so-called acute phase reactants, including CRP, and also stimulates a shift in the production of cells in the bone marrow so that more PMNs are produced. Therefore, cytokines including TNF, IL-1β and IL-6 are essentially responsible for the features of systemic inflammation and inflammatory status and could be potentially useful as biomarkers of sepsis, SLE and inflammation-associated diseases. Despite that cytokines have been used as powerful biomarkers, efforts to provide more insights to pathophysiology of systemic inflammation or inflammatory diseases and to identify more accurate and sensitive predictors of diagnosis, prognostication or therapy are important. We hypothesize that combination of a panel of cytokines with specific potential metabolites which are associated with progression or development of systemic inflammation will be more likely to yield diagnostic success given the high specificity.

Deng *et al.* isolated from the conditioned medium of human fibroblast soluble small-molecule factors (named cytoguardins) that inhibit COX-2 expression in fibroblasts, macrophages and endothelial cells induced by LPS, proinflammatory cytokines and mitogenic factors (Deng et al., FASEB J 16:1286-1288, 2002). The inventors have recently identified by comparative metabolomics a novel tryptophan metabolite, 5-methoxytryptophan (5-MTP) as a cytoguardin (Cheng et al., Proc Natl Acad Sci U S A 109:13231-13236, 2012). L-tryptophan is an essential amino acid which serves not only as building block of protein synthesis but also as substrate for producing diverse metabolites some of which, such as serotonin, melatonin and kynurenine play well-recognized important physiological roles. 5-MTP is produced from L-tryptophan via a novel pathway (Cheng et al., 2012). 5-MTP inhibits proinflammatory mediator-induced COX-2 expression in fibroblasts and cancer cells and reduces cancer cell migration and invasion and cancer metastasis in a xenograft model (Cheng et al., 2012).

The physiological functions and clinical relevance of 5-MTP remain to be elucidated. Since COX-2 is a major mediator of inflammation and overexpressed in diverse inflammatory disorders including sepsis (Bitto et al., Crit Care 16:R32, 2012; Ejima et al., FASEB J 17:1325-1327, 2003; Wu et al., Arterioscler Thromb Vasc Biol 25:679-685, 2005), we speculate that 5-MTP play an important role inflammatory diseases such as sepsis and systemic lupus erythematosus (SLE). To investigate the role of 5-MTP in systemic inflammation, in this study, we determined the clinical relevance of 5-MTP in patients with sepsis and SLE. US2012/004854 relates to a computer-implemented method of selecting subject for treatment of cancer comprises inputting expression data of panel of serum metabolic biomarkers and determining whether expression of metabolic biomarkers is indicative of cancer; the markers include 5-MTP detected using specific binding assays, such as an ELISA assay.

### SUMMARY OF INVENTION

Accordingly, in one aspect, the present invention provides a diagnostic kit of inflammatory diseases, which comprises an agent for detecting the level of 5-methoxytryptophan (5-MTP) in the serum of a subject.

In certain embodiments of the present invention, the inflammatory disease includes sepsis and systemic lupus erythematosus (SLE).

In one embodiment of the present invention, the detecting agent comprises an anti-5-MTP antibody and a 5-MTP-conjugated HRP compound.

In another aspect, the present invention relates to a diagnostic method of inflammatory diseases, which comprises detecting the reduction in the level of 5-methoxytryptophan (5-MTP) in the serum of a subject.

Preferably, the diagnostic method of the present invention comprises a competitive ELISA to measure serum 5-MTP level.

In certain embodiments of the present invention, the diagnostic method comprises contacting an anti-5-MTP antibody with a mixture of 5-MTP-conjugated HRP and serum sample of the subject.

### BREIF DESCRIPTION OF THE DRAWINGS

Fig. 1A shows the serum 5-MTP concentration in septic patients (n=50) and healthy donors (n=30) measured by comparative 5-MTP ELISA. Fig 1B shows the analysis of the discriminative power 5-MTP, hs-CRP and IL-β in sepsis by ROC curve.
Fig. 2 shows that 5-MTP is depressed by LPS in a murine sepsis model. Serum 5-MTP concentration were measured by comparative 5-MTP ELISA in mice before and 24 h after LPS infusion (n=8). The solid black line denotes mean.
Fig. 3A and 3B show that 5-MTP derived from endothelial cells inhibits LPS-induced COX-2 expression (3A) and cytokine production in macrophages (3B). Fig. 3C shows that 5-MTP neutralizing antibodies dose-dependently abrogated the suppressing effect of HUVEC-CM while a control IgG had no effect.
Fig. 4 shows the serum 5-MTP concentration in SLE patients (n=135) and healthy donors (n=30) measured by comparative 5-MTP ELISA.

### DETAILED DESCRIPTION OF THE INVENTION

The other characteristics and advantages of the present invention will be further illustrated and described in the following examples. The examples described herein are using for illustrations, not for limitations of the invention.

### Development of 5-MTP specific competitive ELISA

It was unknown whether 5-MTP is present in the circulating blood. In this study, we developed a highly specific comparative ELISA to measure serum 5-MTP levels in human patients, healthy control and mouse. A critical step for performing the 5-MTP specific competitive ELISA is generation of 5-MTP-conjugated HRP. The procedures of 5-MTP-conjugated HRP generation are described below: (1) 0.8∼1.0 mg 5-MTP is completely dissolved in 100 µl 1M sodium bicarbonate buffer (pH=8.3) with 30 % DMSO. Add this solution to a tube of NH2-Reactive Peroxidase and pipette to dissolve NH₂-Reactive Peroxidase completely; (2) Pipette and incubate the tube at 37 for 1 h, and then incubated it at 4 for overnight; (3) Add 200 µl PBS to the reaction solution, and transfer the solution to a Filtration Tube; (4) Centrifuge at 8,000 x g for 10 min and then discard the filtrate, add 200 µl PBS to the tube; (5) Centrifuge at 8,000 x g for 10 min and discard the filtrate. Add 200 µl PBS and centrifuge again; (6) Add 400 µl Storage Buffer (PBS with 30 % DMSO), and pipette about 10 times to dissolve the conjugate; (7) Transfer the solution to a microtube, and store at -20 or 80 °C.

Serum 5-MTP is measured by a modified competitive ELISA in a 96-well microtiter plate as described below: (1) 96-well microtiter plate is coated with polyclonal rabbit anti-5-MTP antibodies in a coating buffer, 0.05 M carbonate-bicarbonate (pH 9.6) at 4°C overnight; (2) After PBST washing and treatment with blocking buffer, a mixture of 5-MTP-conjugated HRP and 5-MTP standards or serum samples is added to the wells and incubated at 4 °C overnight; (3) The wells are washed with PBST, and treated with a substrate tetramethylbeuzidine at room temperature for 20∼30 minutes; (4) After incubation, 0.1 N H₂SO₄ stop solution is added to each well; and (5) Within 30 min, the product is analyzed at 450 nm. The calibration curve is established by using pure 5-MTP at concentrations of 0.1-500 µM.

### Determinant of serum 5-MTP level in sepsis patients by competitive ELISA

To determine the clinical relevance of 5-MTP, we measured serum 5-MTP in patients with clinical evidence of sepsis and normal healthy controls. Serum 5-MTP was measured by a modified competitive ELISA in a 96-well microtiter plate coated with polyclonal rabbit anti-5-MTP antibodies (Abcam) using a coated buffer (0.05 M carbonate-bicarbonate, pH 9.6) at 4°C overnight. After PBST washing and treatment with blocking buffer, mixture of 5-MTP-conjugated HRP generated by using an NH2 peroxidase labeling kit (Abnova) and 5-MTP standards or serum samples were added to the wells and incubated at 4°C overnight. The wells were washed and treated with a substrate tetramethylbeuzidine at room temperature for 20∼30 minutes. After then, 0.1 N H₂SO₄ stop solution was added to each well. Within 30 min, the product was analyzed at 450 nm. The calibration curve was established by using pure 5-MTP at concentrations of 0.1-500 µM.

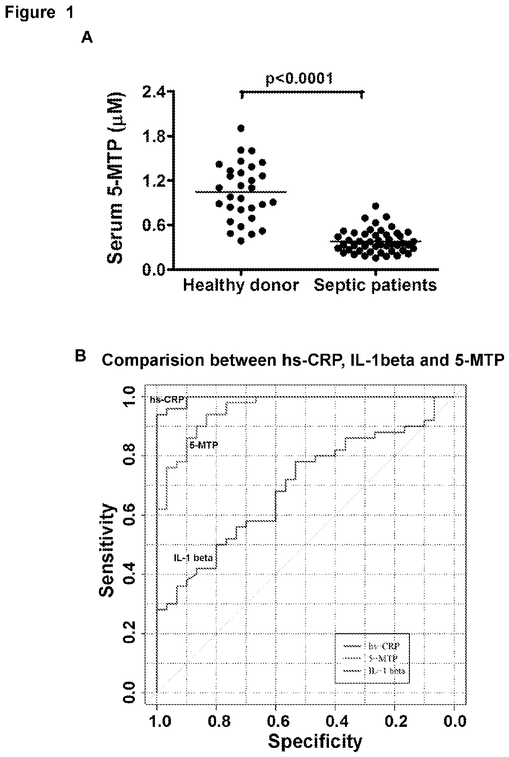

Fifty sepsis patients (twenty-nine male and 21 female with a mean age of 64.0 ± 19.6 years old) were enrolled. Average normal serum 5-MTP level from 30 healthy controls was 1.05 µM (Table1). 5-MTP concentration in sepsis patients was markedly reduced with a mean value of 0.37 µM (Fig. 1A and Table 1). Patients with sepsis had a significant increase in high sensitive-C reactive protein (hs-CRP) (98.2 ± 74.3 mg/L versus 0.5 ± 0.5 mg/L, *P* < 0.001) and IL-1β (1327 ± 2445 pg/ml versus 526 ± 175 pg/ml, *P* = 0.025) level compared with those of healthy subjects (Table I).

**Table I. Serum level of 5-MTP, hs-CRP and IL-1β in septic patients and healthy donors**

| | **Septic patient *n*=50** | **Healthy donors *n*=30** | ***P* value** |
|---|---|---|---|
| **5-MTP (µM)** | 0.37 ± 0.15 | 1.05 ± 0.39 | < 0.0001 |
| **hs-CRP (mg/L)** | 98.2 ± 74.3 | 0.5 ± 0.5 | < 0.001 |
| **IL-1β (pg/ml)** | 1327 ± 2445 | 526 ± 175 | = 0.025 |

As serum hs-CRP and IL-1β levels were reported to be a diagnostic marker of sepsis severity, we used area under the receiver operating characteristic curve (AUROC) to determine the discriminative power of 5-MTP, hs-CRP and IL-1β in this group of patients (Fig. 1B and Table II).

**Table II. Sensitivity and specificity of 5-MTP, hsCRP and IL-1β at optimum diagnostic cut-off values for sepsis**

| | **Cut-off** | **Sensitivity (%)** | **Specificity(%)** |
|---|---|---|---|
| **5-MTP (µM)** | 0.63 | 83 | 94 |
| **hs-CRP (mg/L)** | 2.73 | 100 | 93.9 |
| **IL-1 β (pg/ml)** | 467.29 | 53.3 | 78 |

The AUCROC value of 5-MTP (0.958, 95% CI 0.919-0.997) was comparable with the value of hs-CRP (0.995, 95% CI 0.987-1.000) and significantly greater than that of IL-1β (0.696, 95% CI 0.581-0.810). Cutoff level of hs-CRP and IL-1β with the optimum diagnostic efficiency derived from the AUROC curves were 2.73 mg/L (sensitivity 100%, specificity 93.9%) and 467.29 pg/ml (sensitivity 53.3%, specificity 78%), respectively. Using serum 5-MTP level of 0.63 µM as a cutoff, the sensitivity was 83% and the specificity was 94% (Fig. 1B). These results suggest that 5-MTP is a predictive biomarker of sepsis severity. More importantly, serum 5-MTP in septic patients was inversely correlated with inflammatory markers such as hs-CRP and IL-1β. In addition, serum 5-MTP has a high discrimination power to predict severity of sepsis. Our results suggest that concurrent measurement of hs-CRP and 5-MTP will provide a better prediction of sepsis severity than measurement of hs-CRP or IL-1β alone.

### 5-MTP is depressed by LPS in a murine sepsis model

We suspected that reduction of serum 5-MTP in sepsis patients is due to endotoxemia. To provide evidence for this issue, we analyzed serum 5-MTP in a murine sepsis model, in which mice were infused with LPS. C57BL/6 mice (6-8 wks old) were treated intraperitoneally with saline or with different concentrations of 5-MTP (23.4 or 100 mg/kg) for 30 min before intraperitoneal administration of LPS (60 mg/kg). Animals were monitored for survival and other clinical signs including ruffled fur, lethargy, diarrhea, and body weight loss. Some animals were sacrificed at different times after LPS injection. Blood samples, peritoneal exudates, lungs, and spleens were collected. All mouse experiments were approved by the Institutional Animal Care and Use Committee, National Health Research Institutes.

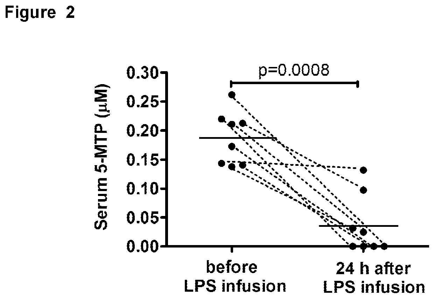

Average serum 5-MTP level in mice prior to LPS treatment was 0.187 µM which is about 1/6 of the mean value in humans. Serum 5-MTP was significantly reduced to 0.036 µM after LPS infusion (Fig. 2). Serum 5-MTP became undetectable or dramatically decreased after LPS infusion in 6 of the eight mice, depressed in 1 and unchanged in the remaining one (Fig. 2). These results suggest that endotoxemia inhibits 5-MTP which contributes to clinical severity.

### 5-MTP derived from endothelial cells inhibits LPS-induced COX-2 expression and cytokine production in macrophages

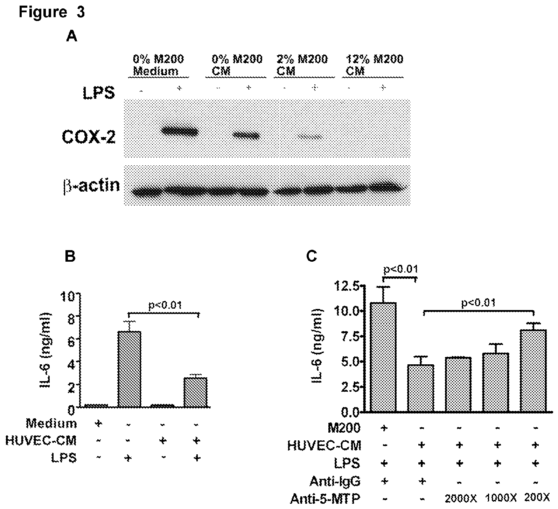

Endothelial cells are considered to play a key role in controlling progression of inflammatory tissue damage in sepsis (Deanfield et al., 2007). We hypothesize that endothelial cells produce soluble factors such as 5-MTP to modulate inflammatory responses. To test this hypothesis, we cultured mouse macrophage RAW264.7 cells with conditioned medium (CM) from HUVECs in the presence or absence of LPS for 24 hours and COX-2 expression and IL-6 production were measured. COX-2 expression and IL-6 production induced by LPS in RAW264.7 cells were attenuated by addition of HUVEC CM (Fig. 3A, B).

As 5-MTP has been identified to be a fibroblast-releasing factor which plays an important role in suppressing proinflammatory mediator induced COX-2 expression (Cheng et al., 2012), we determined whether 5-MTP is a soluble factor in the HUVEC-CM responsible for suppression of LPS-induced COX-2 expression and cytokine production by using 5-MTP neutralizing antibodies. 5-MTP neutralizing antibodies dose-dependently abrogated the suppressing effect of HUVEC-CM while a control IgG had no effect (Fig. 3C).

### SLE patients have a lower level of 5-MTP

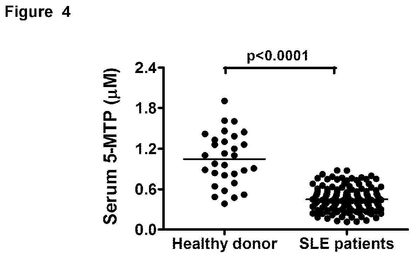

It was unknown whether serum 5-MTP level is associated with SLE pathology. In this study, we also measured serum 5-MTP levels in SLE patients by a highly specific ELISA as described above. 135 patients were enrolled. It is of interests to observe that serum 5-MTP concentration in SLE patients was markedly reduced with a mean value about 0.45 µM (Fig. 4), suggesting that reduction of 5-MTP may compromise the defense against excessive inflammatory responses in SLE.

In summary, it is observed that serum 5-MTP concentrations in septic patients and SLE patients were significantly reduced to about 30 % of that of healthy subjects. More importantly, serum 5-MTP in septic patients was inversely correlated with inflammatory markers such as hs-CRP and IL-1β. In addition, serum 5-MTP has a high discrimination power to predict severity of sepsis. Our results suggest that concurrent measurement of hs-CRP and 5-MTP will provide a better prediction of sepsis severity than measurement of hs-CRP or IL-1β alone. In addition, we also claim that 5-MTP is a novel diagnostic biomarker for predicting sepsis, SLE and inflammatory diseases. Further, the highly specific 5-MTP competitive ELISA acts as a diagnostic kit for gauging occurrence and severity of inflammatory diseases.

## Claims

1. A 5-methoxytryptophan (5-MTP)-specific competitive ELISA kit for the diagnosis of an inflammatory disease, comprising a detecting agent comprising an anti-5-MTP antibody and a 5-MTP-conjugated HRP for detecting the level of 5-MTP in a serum sample of a subject.

2. The 5-MTP-specific competitive ELISA kit of claim 1, wherein the inflammatory disease is sepsis.

3. The 5-MTP-specific competitive ELISA kit of claim 1, wherein the inflammatory disease is systemic lupus (SLE).

4. The 5-MTP-specific competitive ELISA kit of any one of claims 1-3, wherein the anti-5-MTP antibody is for coating on a detecting substrate and the 5-MTP-conjugated HRP is for competing with the 5-MTP in the serum sample in the binding of the anti-5-MTP antibody coated on the detecting substrate.

5. The 5-MTP-specific competitive ELISA kit of any one of claims 1-4, further comprising an agent for detecting the level of high sensitive-C reactive protein (hs-CRP) in a serum sample of a subject.

6. Use of the 5-MTP-specific competitive ELISA kit of any one of claims 1-5 in the diagnosis of an inflammatory disease.

7. Use of the 5-MTP-specific competitive ELISA kit of any one of claims 1-5 in the diagnosis of sepsis.

8. Use of the 5-MTP-specific competitive ELISA kit of any one of claims 1-5 in the diagnosis of systemic lupus (SLE).

## Patentansprüche

1. Ein 5-Methoxytryptophan (5-MTP)-spezifisches kompetitives ELISA-Kit zur Diagnose einer entzündlichen Krankheit, umfassend ein Nachweismittel, welches einen Anti-5-MTP-Antikörper und ein 5-MTP-konjugiertes HRP umfasst, zum Nachweisen des Gehalts von 5-MTP in einer Serumprobe eines Subjektes.

2. Das 5-MTP-spezifische kompetitive ELISA-Kit nach Anspruch 1, wobei die entzündliche Krankheit Sepsis ist.

3. Das 5-MTP-spezifische kompetitive ELISA-Kit nach Anspruch 1, wobei die entzündliche Krankheit systemischer Lupus (SLE) ist.

4. Das 5-MTP-spezifische kompetitive ELISA-Kit nach einem der Ansprüche 1-3, wobei der Anti-5-MTP-Antikörper zum Beschichten eines Nachweissubstrats ist und das 5-MTP-konjugierte HRP mit dem 5-MTP in der Serumprobe um die Bindung des Anti-5-MTP-Antikörpers, welcher auf das Nachweissubstrat aufgebracht ist, konkurriert.

5. Das 5-MTP-spezifische kompetitive ELISA-Kit nach einem der Ansprüche 1-4, ferner umfassend ein Mittel zum Nachweisen des Gehalts von hochsensitivem C-reaktivem Protein (hs-CRP) in einer Serumprobe eines Subjekts.

6. Verwendung des 5-MTP-spezifischen kompetitiven ELISA-Kits nach einem der Ansprüche 1-5 in der Diagnose einer entzündlichen Krankheit.

7. Verwendung des 5-MTP-spezifischen kompetitiven ELISA-Kits nach einem der Ansprüche 1-5 in der Diagnose von Sepsis.

8. Verwendung des 5-MTP-spezifischen kompetitiven ELISA-Kits nach einem der Ansprüche 1-5 in der Diagnose von systemischem Lupus (SLE).

## Revendications

1. Kit ELISA compétitif spécifique au 5-méthoxytryptophane (5-MTP) pour le diagnostic d'une maladie inflammatoire, comprenant un agent de détection comprenant un anticorps anti-5-MTP et une HRP conjuguée au 5-MTP pour détecter le taux de 5-MTP dans un échantillon de sérum d'un sujet.

2. Kit ELISA compétitif spécifique au 5-MTP selon la revendication 1, dans lequel la maladie inflammatoire est la septicémie.

3. Kit ELISA compétitif spécifique au 5-MTP selon la revendication 1, dans lequel la maladie inflammatoire est le lupus érythémateux disséminé (LED).

4. Kit ELISA compétitif spécifique au 5-MTP selon l'une quelconque des revendications 1 à 3, dans lequel l'anticorps anti-5-MTP est destiné à être appliqué sur un substrat de détection et l'HRP conjuguée au 5-MTP est destinée à être en compétition avec le 5-MTP dans l'échantillon de sérum dans la liaison de l'anticorps anti-5-MTP appliqué sur le substrat de détection.

5. Kit ELISA compétitif spécifique au 5-MTP selon l'une quelconque des revendications 1 à 4, comprenant en outre un agent pour détecter le taux de protéine C-réactive hautement sensible (hs-CRP) dans un échantillon de sérum d'un sujet.

6. Utilisation du kit ELISA compétitif spécifique au 5-MTP selon l'une quelconque des revendications 1 à 5 dans le diagnostic d'une maladie inflammatoire.

7. Utilisation du kit ELISA compétitif spécifique au 5-MTP selon l'une quelconque des revendications 1 à 5 dans le diagnostic de la septicémie.

8. Utilisation du kit ELISA compétitif spécifique au 5-MTP selon l'une quelconque des revendications 1 à 5 dans le diagnostic du lupus érythémateux disséminé (LED).
